(19) Europäisches Patentamt
European Patent Office
Office européen des brevets

(11) Publication number: 0 004 410
B1

(12) # EUROPEAN PATENT SPECIFICATION

(45) Date of publication of patent specification: 09.12.81

(51) Int. Cl.³: C 07 C 11/12, C 07 C 2/38, B 01 J 31/28

(21) Application number: 79200142.2

(22) Date of filing: 22.03.79

(54) **A process for preparing 1,7-octadiene.**

(30) Priority: 27.03.78 US 890116
05.03.79 US 16211

(43) Date of publication of application:
03.10.79 Bulletin 79/20

(45) Publication of the grant of the European patent:
09.12.81 Bulletin 81/49

(84) Designated Contracting States:
DE FR GB NL

(56) References cited:
GB - A - 1 341 324
US - A - 3 732 328
US - A - 3 823 199

JOURNAL OF ORGANOMETALLIC CHEMISTRY, vol. 55, 16th July 1973, Lausanne, P. ROFFIA et al.: "Catalysis by palladium salts. IV Selective hydrogenation with formic acid in the palladium catalysed dimerisation of 1,3-butadiene; Synthesis of 1,7-octadiene" * Pages 405—407 *

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Nozaki, Kenzie
1407 Lakecliff
Houston Texas 77077 (US)

(74) Representative: Keuzenkamp, Abraham et al,
c/o Shell Internationale Research Maatschappij
B.V. P.O. Box 302
NL-2501 CH 's-Gravenhage (NL)

EP 0 004 410 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# A process for preparing 1,7-octadiene

The present invention is concerned with a process for preparing 1,7-octadiene by hydrodimerizing butadiene in the presence of solubilized palladium a tertiary phosphine, formic acid, a base and optionally a solvent.

Linear dimerization of butadiene (1,3-butadiene) provides a source of $C_8$-unsaturated hydrocarbon intermediates useful for the synthesis of di-acids, di-esters, diols or diamines. A particularly preferred dimer is 1,7-octadiene which has terminal double bonds and allows the production of product having only terminal functional groups.

It is known to prepare mixtures of octadienes, e.g., 1,6- and 1,7-octadienes, by contacting 1,3-butadiene with solubilized palladium in the presence of a reducing agent, such as formic acid. Solvents may be present which may be non-polar, e.g., see U.S. Patent Specification No. 3,823,199 or polar, e.g., see U.S. Patent Specification 3,732,328, including amines, e.g., see U.S. Patent Specification No. 1,341,324. Tertiary phosphines, such as triphenyl phosphine, may also be present, e.g., see U.S. Patent Specification No. 3,732,328 and Journal of Organometallic Chemistry, 55 (1973), pages 405—407. Organic bases may also be present, e.g., see Tetrahedron Letters No. 2, 1972, pages 163—164.

The Applicants have now surprisingly found that the rate of formation of 1,7-octadiene is increased if the hydrodimerization of 1,3-butadiene, in the presence of solubilized palladium, a tertiary phosphine, formic acid, a base and optionally a solvent, is also carried out in the presence of a supported palladium, platinum and/or rhodium catalyst. Increased rates are important to commercial ventures. High selectivities and conversions at low rates can make a venture unprofitable. The present invention provides significantly enhanced rates over those of the prior art.

Accordingly, the present invention is concerned with a process for preparing 1,7-octadiene by hydrodimerizing butadiene in the presence of solubilized palladium itself or a solubilized palladium compound, complexed with a tertiary phosphine ligand, a tertiary mono-phosphine, formic acid, a base which can neutralize formic acid and optionally an organic solvent, characterized in that a co-catalyst is also present comprising a metal catalyst supported on an inert carrier wherein the supported metal is palladium, platinum or rhodium or mixtures thereof in an amount of from 0.0001 to 30% by weight of the total supported catalyst and wherein the mole ratio of the metal in the supported catalyst to the solubilized palladium itself or the solubilized palladium compound, complexed with a tertiary phosphine ligand ranges from 0.001 to 10. The addition of the supported catalyst to the solubilized palladium tertiary phosphine complex conventionally utilized provides for a significant increase in the rate of conversion of butadiene to 1,7-octadiene as compared to rates utilizing solubilized palladium alone or supported palladium alone.

Solvents are not essential to the process of this invention, but a good organic solvent can promote the rate of reaction by a factor of two or more.

The non-polar solvents disclosed in U.S. Patent Specification No. 3,823,199, such as paraffinic, cycloparaffinic or aromatic solvents are useful in the process of this invention. The solvent can be a paraffin or cycloparaffin containing 5 to 16 carbon atoms, such as hexane, dodecane, pentadecane, cyclohexane, methylcyclohexane and the like. Suitable solvents also include aromatic hydrocarbons, such as benzene, lower alkyl-substituted aromatic hydrocarbons, such as toluene, m-, p- and o-xylene, halogenated aromatic hydrocarbons including chloro-, bromo- and iodo-substituted, such as chlorobenzene and the like. Halogenated lower aliphatic compounds, such as chloroform, methylene chloride, carbon tetrachloride and the like may be used.

Further useful solvents are the amine solvents disclosed in U.K. Patent Specification No. 1,341,324. A wide range of amines are useful provided that they are liquid under reaction conditions. Tertiary amines are preferred to primary and secondary amines. Suitable amine solvents include alkylamines, cycloalkylamines, arylamines and heterocyclic amines, such as morpholine, pyridine, piperazine and piperidine. Examples of these classes of amines are the lower alkylamines containing 2 to 6 carbon atoms in each alkyl group, such as triethylamine; mono-cyclohexylamine, and N-alkylcyclohexylamines containing up to 12 carbon atoms; aniline and N-alkylanilines containing up to 12 carbon atoms and N-alkylmorpholines containing up to 12 carbon atoms. The preferred amine solvent is pyridine.

Further useful solvents are those of moderate co-ordinating ability, and which include nitriles, such as lower alkyl nitriles, hydrocarbon aromatic nitriles including acetonitrile, benzonitrile and the like, amides including benzamide, acetamide, mono- and di-substituted amides where the substituent is preferably lower alkyl. Suitable substituted amides include N-methyl acetamide, N,N-dimethyl acetamide and dimethyl formamide. Di-alkyl sulphoxides, such as dimethyl sulphoxide and sulphones, such as sulfolane and alkyl-substituted sulfolane are satisfactory. Simple ethers, such as the di(lower alkyl) ethers including dimethyl ether, diethyl ether, and the like function satisfactorily. Hydrocarbon aromatic ethers, such as the lower alkyl phenyl ethers may also be used, and include methyl phenyl ether (anisole), ethylphenyl ether (phenetole) and the like. Cyclic, saturated hydrocarbon ethers, such as tetrahydrofuran, tetrahydropyran and the like are also suitable solvents. Lower alkyl diethers, such as

dimethoxy ethane,and the like may be used. In addition, the cyclic diethers, such as 1,4-dioxane are also suitable solvents.

Simple lower alkyl esters of lower alkanoic acids, such as ethyl acetate, methyl acetate, methyl butyrate and the like as well as cyclic diesters, such as ethylene carbonate and propylene carbonate are also suitable solvents of moderate co-ordinating ability. Ketones, including lower aliphatic ketones, such as methyl ethyl ketone and hydrocarbon aromatic ketones, such as acetophenone are also satisfactory solvents. Lower mono- and di-alkanols, such as isopropanol, ethylene glycol and the like may be used, if desired. The preferred solvents of moderate co-ordinating ability include nitriles, formamides, such as dimethylformamide, di(lower alkyl)ethers, lower alkyl phenyl ethers, simple lower alkyl esters of lower alkanoic acids, ketones and lower alkanols.

Particularly useful solvents include pyridine, benzene, dimethyl formamide, chlorobenzene, anisol, N,N-dimethyl acetamide, nitromethane, ethyl acetate, isopropanol, benzonitrile, chloroform, methyl ethyl ketone, acetonitrile, diethyl ether, acetophenone, toluene, ethylene glycol, ethylene carbonate, propylene carbonate and sulfolane. Particularly desired solvents are pyridine, nitromethane, ethylene carbonate and propylene carbonate.

The preferred organic solvents will have from 1 to 20 carbon atoms per molecule. Particularly desired solvents are those which give two-phase systems which allow easy product separation, such as, for example, nitromethane, ethylene carbonate and propylene carbonate.

The amount of solvent added should be sufficient to dissolve the palladium compound-tertiary phosphine complex.

The formic acid is utilized as a source of hydrogen for the process. It is present in the reaction mixture as a salt of the base promoter utilized. It is thought that dissociation of the formic acid-base salt provides a suitable amount of formic acid necessary to provide the required hydrogen. Excess free acid present in the reaction mixture has an inhibitory effect on the reaction.

It is desirable that some formic acid, as the salt, be present during the entire course of the reaction. When operating the process batchwise, this can be accomplished by adding a stoichiometric amount of formic acid initially, 1 mol. of formic acid for every 2 moles of butadiene, or by continuously or periodically adding additional amounts of formic acid. It is highly desirable, however, that the mole ratio of base to formic acid present in the reaction medium never be less than 1.

The base must be one which can neutralize formic acid according to the reaction:

$$HCOOH + B \rightarrow HCOO^{-}HB^{+}$$

The base may be either insoluble or soluble in the reaction medium.

The base may be organic or inorganic. Suitable organic bases typically have dissociation constants greater than $10^{-8}$ and include tertiary amines, such as triethyl amine, tributyl amine, dimethylethyl amine, lutidine, tripropyl amine, N-methyl morpholine, isoquinoline, N-methyl-2,2,6,6-tetramethyl piperidine, 2,8-(dimethylamine)naphthalene and the like.

Suitable inorganic bases include ammonia, the hydroxide bases, such as sodium hydroxide, potassium hydroxide, calcium hydroxide; ammonium hydroxide; the carbonates and bicarbonates, such as sodium carbonate, sodium bicarbonate, potassium carbonate, potassium bicarbonate, calcium carbonate and the like; the weak bases, such as sodium acetate, potassium acetate, ammonium carbonate, ammonium acetate and the like. When the inorganic bases are utilized, small amounts of water may be present.

The mole ratio of base to formic acid should be at least equal to 1. When organic bases are utilized, excess base may be utilized as a solvent of the amine-base salt may be used as the solvent.

The conventional catalyst used in the process of this invention is solubilized palladium, i.e., solubilized palladium itself or a solubilized palladium compound, complexed with a tertiary phosphine ligand. The palladium may be in any of its possible valence states, e.g., 0, +2, etc. Suitable palladium compounds include the palladium carboxylates, particularly palladium carboxylates derived from alkanoic acids containing up to six carbon atoms, such as palladium acetate, complexes, such as palladium acetyl acetonate, bis-benzonitrile palladium(II) and lithium palladous chloride as well as the palladium halides, nitrates and sulphates, such as palladous chloride and palladium nitrate ($Pd(NO_3)_2(OH)_2$) and palladium sulphate. Suitable palladium-phosphine complexes are $Pd(R_3P)_2$ and $Pd(R_3P)_3$. The solubilized palladium itself or the solubilized palladium compound, complexed with a tertiary phosphine ligand is present in the reaction mixture in catalytic amounts; preferably from about 1 to $10^{-8}$ molar and more preferably from 1 to $10^{-6}$ molar.

Any tertiary phosphine which can be dissolved in the reaction solvent may be used. The bis-phosphines, such as 1,3-bis-phenylphosphinopropane and 1,4-bis-diphenylphosphinobutane, will not function in the present invention as the tertiary phosphine, the butadiene conversions obtained are unsatisfactory. Accordingly, monophosphines are used. Suitable mono-phosphines are represented by the formula:

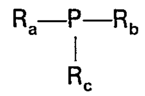

wherein $R_a$, $R_b$ and $R_c$ may be the same or different and are selected from aryl, such as phenyl, p-tolyl, o-tolyl, m-tolyl, p-chlorophenyl, phenoxy, p-methylphenoxy, p-anisoyl, m-anisoyl and the like, alkyl of 1 to 8 carbon atoms, preferably 1 to 5 carbon atoms, alkoxy having from 1 to 8 carbon atoms, but preferably from 1 to 3 carbon atoms. Preferably, $R_a$, $R_b$ and $R_c$ represent aryl, alkyl or a mixture thereof. The more preferred tertiary phosphines of the above formula are the triaryl and trialkyl phosphines. However, the more preferred tertiary phosphines have the general formula:

$$R_1R_2R_3P$$

wherein $R_1$ is an aralkyl or a cycloalkyl group or a branched alkyl or branched alkenyl group having from 3 to 10 carbon atoms with branching occurring at a carbon atom no more than two carbon atoms from the phosphorus atom and $R_2$ and $R_3$ are $R_1$ or independently are alkyl groups having from 1 to 10 carbon atoms, alkenyl groups having up to 10 carbon atoms or aryl groups having up to 10 carbon atoms.

By branched is meant that the alpha- and beta-carbon atoms relative to the phosphorus atom, of the alkyl group may not both be $-CH_2$-linkages. Hydrocarbyl groups are preferred. Illustrative of the $R_1$ moiety are, for branched alkyl, isopropyl, sec.-butyl, tert.-butyl, isobutyl, neopentyl, sec.-pentyl, tert.-pentyl, 2-methylbutyl, sec.-hexyl, tert.-hexyl, 2,2-dimethylpropyl; for aralkyl, benzyl, alpha-methylbenzyl, alpha,alpha-dimethylbenzyl, alpha-methyl-alpha-ethylbenzyl, phenylethyl, phenylisopropyl, phenyl-tert.-butyl; for branched alkenyl, crotyl, methallyl, 1-methylethenyl, 1-methyl-2-propenyl, 1,1-dimethyl-2-propenyl, 1-methyl-3-butenyl; and for cycloalkyl, cyclopropyl, cyclobutyl, cyclopentyl, cyclohexyl, cycloheptyl, and the like.

Illustrative of the $R_2$ and $R_3$ radicals are, for example, methyl, ethyl, propyl, butyl, pentyl, hexyl, octyl, nonyl and decyl for alkyl; allyl, crotyl and methallyl for alkenyl, and phenyl, tolyl, xylyl, ethylphenyl, propylphenyl for aryl. The preferred phosphine is tri(isopropyl)phosphine. Two or more of the instant phosphines may be used in the same reaction or another phosphine may be replaced by reacting in situ one of the instant phosphines. The mole ratio of tertiary mono-phosphine to solubilized palladium itself of the solubilized palladium compound, complexed with a tertiary phosphine ligand is, preferably, at least 1, more preferably ranges from 1:1 to 20:1 and particularly from 2:1 to 5:1. The use of the phosphines of the invention provides extremely high selectivities to 1,7-octadiene.

The promotive supported co-catalyst of the invention comprises a metal selected from the group consisting of palladium, platinum, rhodium or mixtures thereof supported on an inert support. The support employed in these co-catalysts in its broadest aspects is selected from the large number of conventional, porous catalyst carriers or support materials which are essentially inert, under reaction conditions. Such conventional materials may be of natural or synthetic origin. Very suitable supports comprise those of siliceous, aluminous, and carbonaceous compositions. Specific examples of suitable supports are the alumina oxides (including the materials sold under the trade name "Alundum"), charcoal, activated carbon, pumic, magnesia, zirconia, kieselguhr, fuller's earth, silicon carbide, calcium carbonate, porous agglomerates comprising silicon and/or silicon carbide, silica, mullite, selected clays, artificial and natural zeolites and ceramics. Preferred supports are the aluminas, the silicas, and the carbons, such as activated charcoal and graphite.

The co-catalysts are prepared in a conventional manner by impregnating the support with a solution of a suitable metal compound, and then heating the impregnated support in a reducing environment to reduce the metal compound to the metal.

Suitable impregnating solutions are made, for example, from metal carboxylates, such as acetates, halides, nitrates, sulphates and the like. Typical examples are aqueous solutions of palladous chloride, palladous nitrate, palladous acetate, platinum chloride chloro-platinic acid, rhodium nitrate and the like.

The co-catalysts utilized in the invention are not novel per se and find use to-day in many industries, especially the petrochemical industries, for the hydrogenation and dehydrogenation of organic compounds. The supported metal catalysts are readily available commercially.

Catalysts according to this invention contain from 0.001 to 30, preferably from 0.001 to 15, more preferably from 0.001 to 10, and most preferably from 0.001 to 1 per cent by weight of metal based on the total weight of the catalyst.

The mole ratio of the metal in the supported catalyst to the solubilized palladium itself or the solubilized palladium compound, complexed with a tertiary phosphine ligand ranges from 0.001 to 10, preferably from 0.001 to 3, and more preferably from 0.001 to 1.

The supported co-catalyst may be used in bulk or powdered form. The powdered form (less than about 100 mesh U.S. sieve series) is useful in a batch reaction utilizing a homogeneous conventional palladium-phosphine complex catalyst. In continuous processes the co-catalyst may be used as pellets, rings or the like in a fixed bed in conjunction with a homogeneous conventional palladium-phosphine complex catalyst or may be admixed in a fixed bed with a heterogeneous palladium phosphinated resin complex.

The addition of carbon dioxide to the reaction system has been found to increase the extent of butadiene conversion, but does not affect the selectivity. When it is desired to use carbon dioxide to increase the conversion rate, the partial pressure of the $CO_2$ in the reaction system may be from about

10 to about 100 psia. Since carbon dioxide is a by-product of the process, it is possible to generate sufficient carbon dioxide *in situ* to enhance the conversion rates.

The process can be either continuous or batch. The reaction temperature of the process is not critical, however, it is preferred to maintain the reaction between about 0 and about 100°C, preferably between about 20 and about 70°C. The process is conducted under a sufficient pressure to maintain liquid phase conditions at the reaction temperature. Typically the pressure is autogenous.

The process of this invention is particularly useful when applied to a butadiene/isobutene/n-butenes (BBB) stream from an oil pyrolysis unit. These BBB streams are the $C_4$ cut from a thermal cracking unit typically containing 30—40% butadiene, 20—35% isobutene and 20—30% n-butenes and many minor components including about $\frac{1}{2}$% of vinyl acetylene. Vinyl acetylene is a moderate retarder for butadiene hydrodimerization. The addition of from about 0.2 to about 4% by volume of hydrogen along with the supported metal co-catalyst of this invention to the BBB hydrodimerization system eliminates the retardation, presumably by hydrogenating the vinylacetylene.

The invention will now be illustrated by reference to the following Examples.

## Examples 1 to 3

To an 80 ml glass-lined autoclave were charged 2.7 x $10^{-5}$ moles of palladium as a 10% water solution of $Pd(NO_3)_2(OH)_2$, 5.4 x $10^{-5}$ moles (isopropyl)$_3$phosphine, 2.5 grams of triethylamine formic acid salt, 10 ml of pyridine and 2 g of 1,3-butadiene. The stirred reactor was heated to 45°C for 1 hour, cooled and the product was analyzed by gas chromatography for the amount of 1,7-octadiene present. The result is given in Table I as Example a.

The above experiment was repeated three additional times using 0.0001 g, 0.001 g and 0.01 g of 10%w palladium on carbon catalyst (Baker and Co., Inc.) in addition to the solubilized catalyst. The results are given in Table I as Examples 1—3. Column 4 of the Table gives the average rate (R) of formation of 1,7-octadiene in the product per hour. Column 5 is the rate (R) normalized to the rate obtained using solubilized palladium alone ($R_o$), and column 6 gives the ratio of supported palladium to solubilized palladium.

TABLE I

| | *Moles of Palladium Added* | | Rate, %w | | |
|---|---|---|---|---|---|
| Example | Solubilized | Supported | 1,7-octadiene in Product per hour | Normalized Rate $R/R_o$ | Supported Pd/ solubilized Pd |
| a | 2.7 x $10^{-5}$ | none | 5.87 | 1.0 | — |
| 1 | 2.7 x $10^{-5}$ | 9.4 x $10^{-8}$ | 8.5 | 1.4 | 0.0035 |
| 2 | 2.7 x $10^{-5}$ | 9.4 x $10^{-7}$ | 16.0 | 2.7 | 0.035 |
| 3 | 2.7 x $10^{-5}$ | 9.4 x $10^{-6}$ | 10.9 | 1.9 | 0.35 |
| b | none | 9.4 x $10^{-7}$ | 0.04 | — | — |
| c | none | 9.4 x $10^{-6}$ | 0.33 | — | — |

Examples b and c are repeats of Examples 2 and 3 respectively using only supported palladium alone.

## Examples 4 to 9

To an 80 ml glass-lined autoclave were charged 2.7 x $10^{-5}$ moles of palladium as a 10% water solution of $Pd(NO_3)_2(OH)_2$, 5.4 x $10^{-5}$ moles (isopropyl)$_3$phosphine, 2.5 grams of triethylamine formic acid salt, 10 ml of pyridine and 2 g of 1,3-butadiene and the appropriate supported metal co-catalyst from Table II. The stirred reactor was heated to 45°C for 1 hour, cooled and the product was analyzed by gas chromatography for the amount of 1,7-octadiene present. The results are shown in Table II. All powders used were less than 100 mesh.

TABLE II

| Example | Supported Metal C-Catalyst | Moles of Supported Pd | Rate, %w, 1,7-octadiene in Product per Hour |
|---|---|---|---|
| a | None | — | 5.87 |
| 4 | 1) 0.05 g of 0.2% Pd/C | $9.4 \times 10^{-7}$ | 10.0 |
| 5 | 3) 0.01 g of 10% $Pd/Al_2O_3$ | $9.4 \times 10^{-7}$ | 14.7 |
| 6 | 4) 0.02 g of 5% $Pd/Al_2O_3$ | $9.4 \times 10^{-6}$ | 13.25 |
| 7 | 5) 0.02 g of 5% $Pd/CaCO_3$ | $9.4 \times 10^{-6}$ | 9.58 |
| 8 | 6) 0.1 g of 1% Pt/C | $9.4 \times 10^{-6}$ | 7.79 |
| 9 | 7) 0.02 g of 5% $Rh/Al_2O_3$ | $9.4 \times 10^{-6}$ | 9.41 |
| e | 8) 0.02 g of 5% Ru/C | $9.4 \times 10^{-6}$ | 5.0 |
| f | 9) 0.1 g of 10% Ni/C | $9.4 \times 10^{-5}$ | 5.35 |
| g | 9) 0.01 g of 10% Co/C | $9.4 \times 10^{-6}$ | 5.59 |
| h | 9) 0.01 g of 10% Fe/C | $9.4 \times 10^{-6}$ | 5.92 |

1) 0.2% Pd/Charcoal Matheson, Coleman & Bell

3) 10% $Pd/Al_2O_3$ Engelhard Industries, Inc.

4) 5% $Pd/CaCO_3$ Engelhard Industries, Inc.

5) 5% $Pd/CaCO_3$ Engelhard Industries, Inc.

6) 1% Pt/Charcoal Matheson, Coleman & Bell

7) 5% $Rh/Al_2O_3$ Engelhard

8) 5% Ru/C Engelhard

9) Union Carbide porous carbon carrier ($A > 1000 m^2/gm$) first impregnated with metal nitrate solution, dried and then reduced with hydrogen at 500°C.

The above results demonstrate the superiority of supported palladium, platinum and rhodium when compared to other supported catalysts.

Example 10

To an 80 ml glass-lined autoclave were charged $2.7 \times 10^{-5}$ moles of palladium of the appropriate palladium compound listed in Table III, 5.4 $10^{-5}$ moles of $(isopropyl)_3$phosphine, 2.5 g of triethylamine formic acid salt, 10 ml of pyridine, and 2 g of 1,3-butadiene. The stirred reactor was heated to 45°C for 1 hour, cooled and the product was analyzed by gas chromatography for the amount of 1,7-octadiene present. The above reaction was repeated adding using 0.01 g ($9.4 \times 10^{-6}$ moles) of 10% Pd/C (Baker $<$ 100 mesh). The results are shown in Table III.

TABLE III

| Pd Salt Utilized | Rate, %w 1,7-Octadiene in Product per Hour<br>No Co-Catalyst Utilized | 10% Pd/C Co-Catalyst |
|---|---|---|
| $Pd(NO_3)_2(OH)_2$ (10% aqueous solution) | 5.87 | 16.0 |
| $Pd(NO_3)_2(OH)_2$ | 5.61 | 14.67 |
| $Pd(acetate)_2$ | 0.81 | 4.51 |
| $Pd(acetylacetonate)_2$ | 1.86 | 4.10 |
| $PdSO_4$ | 0.65 | 5.11 |
| $PdCl_2$ | 0.48 | 1.48 |

The above results show that the rate enhancement effect is shown for a number of different solubilized palladium salts.

Example 11

To an 80 ml glass-lined autoclave were charged $0.9 \times 10^{-5}$ moles as a 10% water solution of $Pd(NO_3)_2(OH)_2$, $1.8 \times 10^{-5}$ moles $(isopropyl)_3$phosphine, 2.5 grams of triethylamine formic acid salt, 10 ml of pyridine and 2 g of 1,3-butadiene. The stirred reactor was heated to 45°C for 1 hour, cooled and the process was analyzed by gas chromatography for the amount of 1,7-octadiene present. This Example uses one-third the amount of solubilized palladium used in Example a. The results are shown in Table IV.

The above experiment was repeated three additional times using 0.0001 g, 0.001 g and 0.01 g of 10%w palladium on carbon catalyst (Baker and Co., Inc.) in addition to the solubilized catalyst. The results are shown in Table IV. Column 3 gives the average rate (R) of formation of 1,7-octadiene in the product per hour. Column 4 is the rate (R) normalized in the rate obtained using solubilized palladium alone ($R_o$), and column 5 gives the ratio of supported palladium to solubilized palladium.

TABLE IV

| Moles of Palladium Added | | Rate, %w 1,7-octa-diene in Product per Hour | Normalized Rate R/Ro | Supported Pd/ Solubilized Pd |
|---|---|---|---|---|
| Solubilized | Supported | | | |
| $9 \times 10^{-6}$ | — | 1.70 | 1.0 | — |
| $9 \times 10^{-6}$ | $9.4 \times 10^{-6}$ | 1.80 | 1.06 | 1 |
| $9 \times 10^{-6}$ | $9.4 \times 10^{-7}$ | 3.3 | 1.94 | 0.1 |
| $9 \times 10^{-6}$ | $9.4 \times 10^{-8}$ | 2.7 | 1.59 | 0.01 |

Example 12

To an 80 ml glass-lined autoclave were charged $2.7 \times 10^{-5}$ moles of palladium acetate, $1.08 \times 10^{-4}$ moles of (isopropyl) phosphine, 2.5 g of triethylamine-formic acid salt, 10 millilitres of pyridine and 5 g of a BBB stream containing 40.34% 1,3-butadiene. The stirred reactor was heated to 40°C for 1 hour, cooled and the product was analyzed by gas chromatography for the amount of 1,7-octadiene present, giving a rate of 0.12%w 1,7-octadiene per hour. The above reaction was repeated adding 0.01 g of 10% Pd/C (Baker < 100 mesh) and 2% hydrogen basis butadiene to the reaction mixture. The rate was increased to 3.98%w 1,7-octadiene per hour.

## Claims

1. A process for preparing 1,7-octadiene by hydrodimerizing butadiene in the presence of solubilized palladium itself or a solubilized palladium compound, complexed with a tertiary phosphine ligand, a tertiary mono-phosphine, formic acid, a base which can neutralized formic acid and optionally an organic solvent, characterized in that a co-catalyst is also present comprising a metal catalyst supported on an inert carrier wherein the supported metal is palladium, platinum or rhodium or mixtures thereof in an amount of from 0.0001 to 30% by weight of the total supported catalyst and wherein the mole ratio of the metal in the supported catalyst to the solubilized palladium itself or the solubilized palladium compound, complexed with a tertiary phosphine ligand ranges from 0.001 to 10.

2. A process as claimed in claim 2, characterized in that the support is siliceous, aluminous or carbonaceous in nature.

3. A process as claimed in claim 1 or claim 2, characterized in that the amount of metal is from 0.001 to 15 per cent by weight of the total supported catalyst.

4. A process as claimed in any one of the preceding claims, characterized in that the temperature is from 0°C to 100°C.

5. A process as claimed in any one of the preceding claims, characterized in that the amount of solubilized palladium itself or the solubilized palladium compound, complexed with a tertiary phosphine ligand is from 1 to $10^{-8}$ molar.

6. A process as claimed in any one of the preceding claims, characterized in that the molar ratio of tertiary mono-phosphine to solubilized palladium itself or the solubilized palladium compound, complexed with a tertiary phosphine ligand is at least 1.

7. A process as claimed in any one of the preceding claims, characterized in that the molar ratio of base to formic acid is at least 1.

8. A process as claimed in any one of the preceding claims, characterized in that the mole ratio of metal in the supported catalyst to the solubilized palladium itself or the solubilized palladium compound, complexed with a tertiary phosphine ligand ranges from 0.001 to 3.

9. A process as claimed in claim 8, characterized in that the mole ratio of metal in the supported catalyst to the solubilized palladium itself or the solubilized palladium compound, complexed with a tertiary phosphine ligand ranges from 0.001 to 1.

10. A process as claimed in any one of the preceding claims, characterized in that the tertiary mono-phosphine is of the general formula:

$$R_1R_2R_3P$$

wherein $R_1$ is an aralkyl or a cycloalkyl group or a branched alkyl or branched alkenyl group having from 3 to 10 carbon atoms with branching occurring at a carbon atom no more than two carbon atoms from the phosphorus atom and $R_2$ and $R_3$ are $R_1$ or independently are alkyl groups having from 1 to 10 carbon atoms, alkenyl groups having up to 10 carbon atoms or aryl groups having up to 10 carbon atoms.

11. A process as claimed in claim 10, characterized in that the tertiary mono-phosphine is tri(isopropyl)phosphine.

12. A process as claimed in any one of the preceding claims, characterized in that the base is a tertiary amine.

13. A process as claimed in any one of the preceding claims, characterized in that the solubilized palladium is palladium nitrate.

14. A process as claimed in any one of the preceding claims, characterized in that the butadiene is contained in a butadiene/isobutene/n-butenes stream from an oil pyrolysis unit and from about 0.2 to about 4 per cent. by weight of hydrogen is added to the reaction mixture.

## Revendications

1. Procédé pour la préparation de 1,7-octadiène en hydrodimérisant du butadiène en présence de palladium solubilisé ou d'un composé du palladium solubilisé, complexé avec un ligand phosphine tertiaire, d'une mono-phosphine tertiaire, d'acide formique, d'une base qui peut neutraliser l'acide formique et éventuellement d'un solvant organique, caractérisé en ce qu'un cocatalyseur est également présent, comprenant un métal catalytique déposé sur un support inerte, le métal supporté étant du palladium, du platine ou du rhodium ou leurs mélanges à raison de 0.0001 à 30% en poids par rapport au catalyseur supporté total et le rapport molaire du métal dans le catalyseur supporté au palladium solubilisé ou au composé du palladium solubilisé, complexé avec un ligand phosphine tertiaire, étant compris entre 0,001 et 10.

2. Procédé selon la revendication 1, caractérisé en ce que le support est de nature siliceuse, alumineuse ou carbonée.

3. Procédé selon la revendication 1 ou 2, caractérisé en ce que la quantité de métal est comprise entre 0,001 et 15 pour cent en poids par rapport au catalyseur supporté total.

4. Procédé selon l'une des revendications précédentes, caractérisé en ce que la température est comprise entre 0°C et 100°C.

5. Procédé selon l'une des revendications précédentes, caractérisé en ce que la quantité molaire de palladium solubilisé ou de composé du palladium solubilisé, complexé avec un ligand phosphine tertiaire, est comprise entre 1 et $10^{-8}$.

6. Procédé selon l'une des revendications précédentes, caractérisé en ce que le rapport molaire de la monophosphine tertiaire au palladium solubilisé ou au composé du palladium solubilisé, complexé avec un ligand phosphine tertiaire, est d'au moins 1.

7. Procédé selon l'une des revendications précédentes, caractérisé en ce que le rapport molaire de la base à l'acide formique est d'au moins 1.

8. Procédé selon l'une des revendications précédentes, caractérisé en ce que le rapport molaire du métal dans le catalyseur supporté au palladium solubilisé ou au composé du palladium solubilisé, complexé avec un ligand phosphine tertiaire, est comprise entre 0,001 et 3.

9. Procédé selon la revendication 8, caractérisé en ce que le rapport molaire du métal dans le catalyseur supporté au palladium solubilisé ou au composé du palladium solubilisé, complexé avec un ligand phosphine tertiaire, est compris entre 0,001 et 1.

10. Procédé selon l'une des revendications précédentes, caractérisé en ce que la mono-phosphine tertiaire est de la formule générale:

$$R_1R_2R_3P$$

dans laquelle $R_1$ est un groupe aralcoyle ou cycloalcoyle ou un groupe alcoyle ramifié ou alcényle ramifié ayant de 3 à 10 atomes de carbone, la ramification se trouvant à position qui n'est pas placée plus loin que deux atomes de carbone de l'atome de phosphore et $R_2$ et $R_3$ sont tels que $R_1$ ou sont, indépendamment, des groupes alcoyle ayant de 1 à 10 atomes de carbone, des groupes alcényle ayant jusqu'à 10 atomes de carbone ou des groupes aryle ayant jusqu'à 10 atomes de carbone.

11. Procédé selon la revendication 10, caractérisé en ce que la mono-phosphine tertiaire est le tri(isopropyl)phosphine.

12. Procédé selon l'une des revendications précédentes, caractérisé en ce que la base est une amine tertiaire.

13. Procédé selon l'une des revendications précédentes, caractérisé en ce que le palladium solubilisé est du nitrate de palladium.

14. Procédé selon l'une des revendications précédentes, caractérisé en ce que le butadiène est contenu dans un courant de butadiène/isobutène/n-butènes provenant d'une unité de pyrolyse d'huile et que l'on ajoute d'environ 0,2 à environ 4 pour cent en poids d'hydrogène ou mélange réactionnel.

**Patentansprüche**

1. Verfahren zur Herstellung von 1,7-Oktadien durch Hydromerisierung von Butadien in Gegenwart von löslich gemachtem Palladium als solchem oder der löslich gemachten Palladium-verbindung, das oder die mit einem tertiären Phosphinliganden komplexiert worden ist, eines tertiären Monophosphins, Ameisensäure, einer Base, die Ameisensäure neutralisieren kann sowie gegebenenfalls einem organischen Lösungsmittel, dadurch gekennzeichnet, dass auch noch ein Co-Katalysator anwesend ist, welcher einen Metallkatalysator auf einem inerten Träger umfasst, wobei das auf dem Träger befindliche Metall Palladium, Platin oder Rhodium oder ein Gemisch von diesen ist, und zwar in Mengen von 0,0001 bis 30 Gewichtsprozent, bezogen auf den gesamten auf dem Träger befindlichen Katalysator, und das molare Verhältnis des Metalls in dem auf dem Träger befindlichen Katalysator zu dem löslich gemachten Palladium als solchem oder der löslich gemachten Palladiumverbindung, das oder die mit einem tertiären Phosphinliganden komplexiert worden ist, 0,001 bis 10 beträgt.

2. Verfahren nach Anspruch 1, dadurch gekennzeichnet, dass der Träger von kieselsäurealuminium- oder kohlenstoffhaltiger Art ist.

3. Verfahren nach Anspruch 1 oder 2, dadurch gekennzeichnet, dass der Metallgehalt 0,001 bis 15 Gewichtsprozent des gesamten auf dem Träger befindlichen Katalysators ausmacht.

4. Verfahren nach einem der vorhergehenden Ansprüche, dadurch gekennzeichnet, dass die Temperatur 0°C bis 100°C beträgt.

5. Verfahren nach einem der vorhergehenden Ansprüche, dadurch gekennzeichnet, dass der molare Anteil an löslich gemachtem Palladium als solchem oder der löslich gemachten Palladiumverbindung, das bzw. die mit einem tertiären Phosphinliganden komplexiert worden ist, 1 bis $10^{-8}$ beträgt.

6. Verfahren nach einem der vorhergehenden Ansprüche, dadurch gekennzeichnet, dass das molare Verhältnis von tertiärem Monophosphin zu löslich gemachtem Palladium als solchem oder der löslich gemachten Palladiumverbindung, das bzw. die mit einem tertiären Phosphinliganden komplexiert worden ist, mindestens 1 beträgt.

7. Verfahren nach einem der vorhergehenden Anprüche, dadurch gekennzeichnet, dass das molare Verhältnis von Base zu Ameisensäure mindestens 1 beträgt.

8. Verfahren nach einem der vorhergehenden Ansprüche, dadurch gekennzeichnet, dass das Molverhältnis des Metalls in dem auf dem Träger befindlichen Katalysator zu dem löslich gemachten Palladium als solchem oder der löslich gemachten Palladiumverbindung, das bzw. die mit einem tertiären Phosphinliganden komplexiert worden ist, 0,001 bis 3 beträgt.

9. Verfahren nach Anspruch 8, gekennzeichnet, dass das Molverhältnis des Metalls in dem auf dem Träger befindlichen Katalysator zu dem löslich gemachten Palladium als solchem oder der löslich gemachten Palladiumverbindung, das oder die mit einem tertiären Phosphinliganden komplexiert worden ist, 0,001 bis 1 beträgt.

10. Verfahren nach einem der vorhergehenden Ansprüche, dadurch gekennzeichnet, dass das tertiäre Monophosphin die allgemeine Formel

$$R_1R_2R_3P$$

aufweist, in der $R_1$ eine Aralkyl- oder eine Cycloalkylgruppe oder eine verzweigte Alkyl- oder verzweigte Alkenylgruppe mit 3 bis 10 Kohlenstoffatomen ist, wobei die Verzweigung an einem Kohlenstoffatom stattfindet, das nicht mehr als zwei Kohlenstoffatome von dem Phosphoratom entfernt ist, und $R_2$ und $R_3$ $R_1$ oder unabhängig voneinander Alkylgruppen mit 1 bis 10 Kohlenstoffatomen, Alkenylgruppen mit bis zu 10 Kohlenstoffatomen oder Arylgruppen mit bis zu 10 Kohlenstoffatomen sind.

11. Verfahren nach Anspruch 10, dadurch gekennzeichnet, dass das tertiäre Monophosphin Tri(isopropyl)phosphin ist.

12. Verfahren nach einem der vorhergehenden Ansprüche, dadurch gekennzeichnet, dass die Base ein tertiäres Amin ist.

13. Verfahren nach einem der vorhergehenden Ansprüche, dadurch gekennzeichnet, dass das löslich gemachte Palladium Palladiumnitrat ist.

14. Verfahren nach einem der vorhergehenden Ansprüche, dadurch gekennzeichnet, dass das Butadien in einem Butadien/Isobuten/n-Buten-Strom enthalten ist, der einer Einheit zur Pyrolyse von Öl entstammt, und dass dem Reaktionsgemisch etwa 0,2 bis etwa 4 Gewichtsprozent Wasserstoff zugesetzt werden.